# EUROPEAN PATENT APPLICATION

(11) **EP 3 949 843 A1**
(43) Date of publication of application: **09.02.2022**
(21) Application number: 19928855.6
(22) Date of filing: 14.08.2019
(51) Int. Cl.: A61B 5/04

(54) **PRECISE STEP COUNTING METHOD USING HUMAN BODY CAPACITANCE**

(30) Priority: 12.05.2019 CN 201910391402
(71) Applicant: Wang, Yongfang, Xi'an, Shaanxi 719000 (CN)
(72) Inventor: Wang, Yongfang, Xi'an, Shaanxi 719000 (CN)
(74) Representative: Sun, Yiming
(86) International application number: PCT/CN2019/100467
(87) International publication number: WO 2020/228157

(57) **Abstract**

Disclosed is a precise step counting method using human body capacitance, relating to the field of electronic communications, and in particular to a method for implementing precise step counting using human body capacitance. At present, a wearable intelligent device having a step counting function measures a number of steps by means of an inertial sensor, but has limited precision in a measurement result. Due to the complexity of motion of a human body, missed measurement and incorrect measurement often occur in the inertial sensor-based measurement of the number of steps. The recognition and calculation of the number of steps also occupy a large number of hardware resources. In this respect, a precise step counting method based on human body capacitance, a human body's electrostatic field, or a human body surface potential is provided. When a human body is walking, the actions of leg lifting and leg lowering would change human body capacitance, and the number of steps can be conveniently and quickly measured by detecting a change in a physical signal caused by a change in the human body capacitance, such as a change in a voltage signal of human body skin and a change in a frequency. The method implements precise step counting without occupying hardware spaces and resources, has the advantage of low power consumption, and can be embedded into all current wearable intelligent devices that require a step counting function).

## Description

### FIELD OF TECHNOLOGY

The present disclosure relates to the field of electronic communications, and in particular to a method for implementing precise step counting using human body capacitance.

### BACKGROUND

Nowadays, everyone is very concerned about health. It is already a living habit for many people to record a number of steps they walk, whether by smart bracelets, smart watches, or pedometers, or by cell phones. By measuring the number of steps, energy consumption can be estimated for the purpose of health tracking. However, at present, a wearable portable intelligent device having a step counting function measures the number of steps by using data collected by an inertial sensor, and has limited precision in a measurement result of the number of steps based on the inertial sensor due to the complexity of motion of a human body. For example, if a person sits in an automobile, jolts of the automobile cause incorrect judgment of the number of steps by the intelligent device. As another example, if a bracelet wearer puts his hand in his trouser pocket, the number of steps walked is difficult to detect.

### SUMMARY OF The INVENSION

In view of this, unlike a traditional inertial sensor-based method for measuring a number of steps, the present disclosure implements precise measurement of the number of steps using human body capacitance.

The principle is that a human body is a good conductor, with a certain number of charges distributed on body surface skin, so an electric field is formed between the human body and the ground. The electric field may be expressed by a plurality of physical quantities, such as a skin potential, a human body electrostatic field, human body capacitance, etc., The physical principle of the expressions is based on good conductor properties of the human body. The present disclosure uses the human body capacitance to describe the physical principle. The human body capacitance refers to capacitance formed between the human body and the ground. When the human body moves relative to the ground, such as lifting a leg, the distance between the human body and the ground changes, resulting in a change in the human body capacitance. While the human body capacitance changes, that is, while the electrostatic field between the human body and the ground changes, the charges distributed on the human body skin flow or are redistributed on the human body, and between the human body and the ground. The change in the human body capacitance caused by the motion of the human body, such as a change in a current, voltage, frequency or other physical signal caused by the change in the human body capacitance, is detected directly or indirectly to infer the motion of the human body, thereby achieving precise measurement of the number of steps.

Technical solutions of the present disclosure are further described in detail below in conjunction with the accompanying drawings and embodiments.

### BRIEF DESCRIPTION OF DRAWINGS

Fig. 1 shows a human body capacitance or human body electrostatic field model.
Fig. 2 shows a basic information flow implemented in the present disclosure.
Fig. 3 is a circuit structure diagram in an embodiment of the present disclosure.

### DETAILED DESCRIPTIONS OF EMBODIMENTS

To make the technical problems to be solved, technical solutions, and beneficial effects of the present disclosure clearer and more apparent, the present disclosure will be further described in detail below in conjunction with the accompanying drawings and embodiments. It should be understood that the specific embodiments described herein are only used for explaining the present disclosure, rather than limiting the present disclosure.

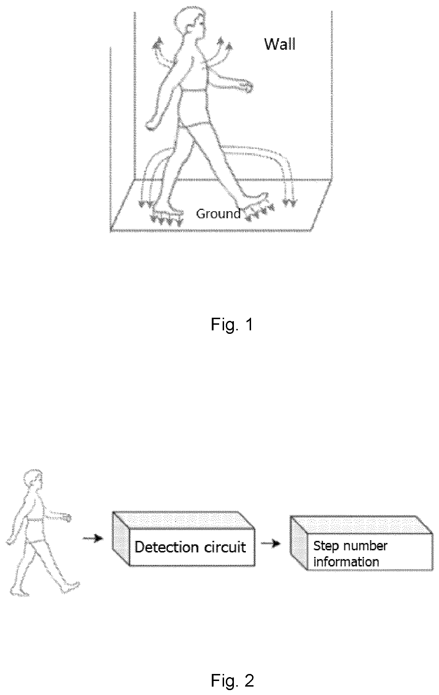

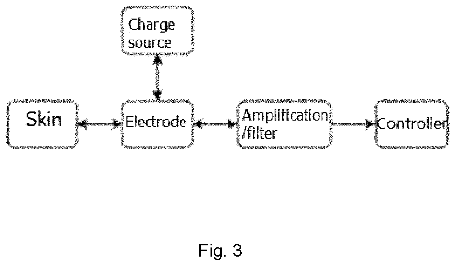

Referring to Figs. 1, 2 and 3, a precise step counting method based on human body capacitance is described in conjunction with the embodiments. It uses an electrode in direct contact with skin or an electrode indirectly coupled to a human body, a charge source, a conventional processing circuit for filtering and amplifying signals, and a controller with analog-to-digital conversion. The controller may also not have an analog-to-digital conversion module, but a separate analog-to-digital converter needs to be added.

In the described example, the human body is directly connected or indirectly coupled to the physical circuit through the electrode. When the human body performs an action of lifting or lowering a leg, the human body capacitance changes, and the change causes redistribution of charges distributed on the human body, and the process is embodied as flow of charges at the electrode.

The flow of the charges may be reflected by a change in a voltage at human body skin. The voltage at the human body skin is detected, and a voltage signal is processed such as being filtered and amplified, to obtain a signal of a skin voltage change when the human body lifts or lowers the leg.

Skin voltage signals thus obtained are counted to obtain a number of times the human body lifts and or lowers legs.

It should be noted that by arranging a capacitor between the electrode and the filter/amplifier circuit, the amplitude and response time of the skin voltage signal change can be adjusted to further improve the precision of detection of the number of steps.

In summary, this embodiment detects the skin voltage signal change caused by the change in the human body capacitance, wherein repetitive capacitance changes produce repetitive skin voltage signal changes, to detect the number of steps of the human body. This example has the characteristics of low power consumption and small space occupation, and can be conveniently embedded into wearable intelligent devices such as intelligent watches and wristbands.

It should be noted that the change in the human body capacitance may also be detected by detecting a change in any other physical signal, such as a frequency or other related human body physical signal in addition to detecting voltage change at the skin.

The preferred embodiments of the present disclosure are described above with reference to the accompanying drawings, but the scope of the claims of the present disclosure is no limited thereto. All modifications, equivalent substitutions and improvement made within the scope and essence of the present disclosure should be encompassed within the scope of the claims of the present disclosure.

## Claims

1. A precise step counting method using human body capacitance, which uses a change in the human body capacitance when a human body lifts and lowers legs to count steps, wherein the method uses the physical phenomenon of the change in the human body capacitance caused by actions of the human body.

2. The step counting method of claim 1, wherein the change in the human body capacitance is also be defined as a change in an electric field between the human body and the environment, or a change in a human body skin potential, both of which are essentially based on good conductor properties of the human body and electrical properties between the human body and the ground.

3. The step counting method of claim 1, wherein when the human body capacitance changes, human body charges are redistributed, and the redistribution of the charges is accompanied by generation of a current; and a number of steps is measured by detecting a change in a current, frequency or other physical signal caused by the change in the human body capacitance.

4. The step counting method of claim 1, wherein when the human body capacitance changes, human body charges are redistributed, and the redistribution of the charges is accompanied by generation of a current; and a change in the current is reflected by measuring a voltage change at skin, i.e., a number of steps is measured by detecting the voltage change at the skin caused by the change in the human body capacitance.

5. The step counting method of claim 1, wherein in addition to counting the steps by measuring a voltage change at skin, the physical quantity of the change in the human body capacitance is reflected by a frequency or other easily measurable physical quantity, to measure a number of steps.

6. The step counting method of claim 1, wherein a conductive electrode is connected or coupled to human body skin, and a charge source, such as a voltage divider circuit, is additionally connected to the electrode to provide a charge source while providing a bias voltage; and the change in the human body capacitance caused by motion is reflected by measuring a voltage change of the electrode, to measure the number of steps.

7. The step counting method of claim 6, wherein a conventional signal processing circuit, such as for filtering and amplification processing, is arranged at a rear end of the electrode, and then analog-to-digital signal acquisition is performed, to improve the precision of measurement of the number of steps.

8. The step counting method of claim 7, wherein the amplitude and response time of a measured signal is adjusted by changing a capacitance value of a capacitor in a filter circuit, or a capacitor additionally arranged between the electrode and the signal processing circuit, to improve the precision of measurement of the number of steps.

9. The step counting method of claim 1, wherein the method achieves low power consumption, does not occupy hardware space or hardware resources, and is convenient to embed into a current wearable intelligent device that requires a step counting function to achieve a step counting function superior to that of an inertial sensor-based step counting method.

## Amended claims

### Amended claims under Art. 19.1 PCT

1. A precise step counting method using human body capacitance, which uses a change in the human body capacitance when a human body lifts and lowers legs to count steps, wherein the method uses the physical phenomenon of the change in the human body capacitance caused by actions of the human body; the human body capacitance refers to capacitance between the human body and the ground; and unlike parasitic capacitance generated by relative motion between silk fabric and a human body described in CN107334189A and CN102300499A, the human body capacitance is an independent, ubiquitous physiological signal that does not rely on a decoration other than the human body such as silk fabric.

2. The step counting method of claim 1, wherein the change in the human body capacitance is also be described as a change in an electric field between the human body and the environment, or a change in a human body skin potential, both of which are essentially based on good conductor properties of the human body and electrical properties between the human body and the ground.

3. The step counting method of claim 1, wherein when the human body capacitance changes, human body charges are redistributed, and the redistribution of the charges is accompanied by generation of a current; and a number of steps is measured by detecting a change in a current, frequency or other physical signal caused by the change in the human body capacitance.

4. The step counting method of claim 1, wherein when the human body capacitance changes, human body charges are redistributed, and the redistribution of the charges is accompanied by generation of a current; and a change in the current is reflected by measuring a voltage change at skin, i.e., a number of steps is measured by detecting the voltage change at the skin caused by the change in the human body capacitance.

5. The step counting method of claim 1, wherein in addition to counting the steps by measuring a voltage change at skin, the physical quantity of the change in the human body capacitance is reflected by a frequency or other easily measurable physical quantity, to measure a number of steps.

Statement under Art. 19.1 PCT
Claim 1 was amended; Claims 2-5 were unchanged; and Claims 6-9 were cancelled.

Claim 1 was amended by adding a difference between the capacitance of the human body and the parasitic capacitance generated by clothing. The step counting methods described in the related patent CN107334189A and other related patent CN102300499A mentioned in the international search report and the written opinion of the international searching authority are based on the parasitic capacitance generated by friction of the silk fabric worn on the human body, which is a different physical signal from the human body capacitance described in the patent of the present disclosure. The human body capacitance is a biophysical signal that does not rely on any external object, but exists in itself, just like human body surface temperature and human body surface humidity.

Claims 6-9 were cancelled because they describe a circuit implementation for measurement of the human body capacitance, which belongs to a technical embodiment, and not claimed.
